# EUROPEAN PATENT SPECIFICATION

(11) **EP 0 967 217 B1**
(45) Date of publication and mention of the grant of the patent: **21.12.2005**
(21) Application number: 99250202.1
(22) Date of filing: 21.06.1999
(51) Int. Cl.: C07F 9/24, C07F 9/6561, C07D 493/10, C07H 21/00, C07F 9/655

(54) **Reagents and methods for solid phase synthesis and display**
Reagenz und Verfahren zu fester Phase Synthese
Réactifs et procédé de synthèse en phase solide

(30) Priority: 22.06.1998 US 102986
(43) Date of publication of application: 29.12.1999
(73) Proprietor: Affymetrix, Inc. (a California Corporation), Santa Clara, CA 95051 (US)
(72) Inventor: McGall, Glenn H., Mountain View, CA 94041 (US); Barone, Anthony D., San Jose, California 95125 (US); Diggelmann, Martin, 4144 Arlesheim (CH)
(74) Representative: UEXKÜLL & STOLBERG

(56) References cited:
- EP-A- 0 523 978
- WO-A-94/10128
- WO-A-95/31434
- WO-A-99/10362
- US-A- 5 143 854
- SHCHEPINOV ET AL: "A versatile reagent for the synthesis of modified oligonucleotides" RUSSIAN JOURNAL OF BIOORGANIC CHEMISTRY,RU,MAIK NAUKA-INTERPERIODICA, vol. 21, no. 2, 6 January 1995 (1995-01-06), pages 107-111, XP002117193 ISSN: 1068-1620
- SMITH T.H. ET AL.: "Oligonucleotide labeling methods 4. Direct labeling reagents with a novel, non-nucleosidic, chirally defined 2-deoxy-beta-D-ribosyl backbone " NUCLEOSIDES & NUCLEOTIDES., vol. 15, no. 10, October 1996 (1996-10), pages 1581-1594, XP002154806 MARCEL DEKKER, INC., US ISSN: 0732-8311
- GUZAEV A ET AL: "SYNTHESIS OF OLIGONUCLEOTIDE ANALOGUES BY USING PHOSPHORAMIDITE REAGENTS DERIVED FROM 2,2-BIS(HYDROXYMETHYL)MALONATES" COLLECTION OF CZECHOSLOVAK CHEMICAL COMMUNICATIONS,GB,ACADEMIC PRESS, LONDON, vol. 61, 1996, pages S226-S229, XP000198778 ISSN: 0010-0765

## Description

### BACKGROUND OF THE INVENTION

The present invention relates to the field of solid phase polymer synthesis. More specifically, the invention provides methods and reagents for solid phase synthesis of oligomer arrays and combinatorial chemistry libraries which may be used, for example, in screening studies for determination of binding affinity or other biological activity.

The synthesis of oligomer arrays and combinatorial libraries of small organic molecules has received considerable attention in both academic and industrial research groups. In part, this attention has resulted from the application of such arrays and libraries to drug discovery or screening to obtain sequence information on unsequenced genes or gene fragments. Many of these applications involve the initial preparation of arrays or libraries on a solid support.

The evolution of solid phase synthesis of biological polymers began with the early "Merrifield" solid phase peptide synthesis, described in Merrifield, *J. Am. Chem. Soc.* 85:2149-2154 (1963). Solid-phase synthesis techniques have also been provided for the synthesis of several peptide sequences on, for example, a number of "pins." See *e.g*., Geysen *et al., J. Immun. Meth*. **102**:259-274 (1987). Other solid-phase techniques involve, for example, synthesis of various peptide sequences on different cellulose disks supported in a column. See Frank and Doring, *Tetrahedron* **44**:6031-6040 (1988). Still other solid-phase techniques are described in U.S. Patent No. 4,728,502 issued to Hamill and WO 90/00626 (Beattie, inventor). Derivatized solid supports having a specific formula for the preparation of a variety of oligonucleotides are disclosed in WO 95/31434 where amongst others a symmetrical compound comprising a disulfide bond is disclosed.

Each of the above techniques produces only a relatively low density array of polymers. For example, the technique described in Geysen *et al*. is limited to producing 96 different polymers on pins spaced in the dimensions of a standard microtiter plate.

Improved methods of forming large arrays of oligonucleotides, peptides and other polymer sequences in a short period of time have been devised. Of particular note, Pirrung *et al*., U.S. Patent No. 5,143,854 (see also PCT Application No. WO 90/15070) and Fodor *et al*., PCT Publication No. WO 92/10092. disclose methods of forming vast arrays of peptides, oligonucleotides and other polymer sequences using, for example, light-directed synthesis techniques. See also, Fodor *et al., Science,* 251:767-777 (1991). These procedures are now referred to as VLSIPS™ procedures.

In the above-referenced Fodor *et al*., PCT application, an elegant method is described for using a computer-controlled system to direct a VLSIPS™ procedure. Using this approach, one heterogenous array of polymers is converted, through simultaneous coupling at a number of reaction sites, into a different heterogenous array. See, U.S. Patent Nos. 5,384,261 and 5,677,195.

The development of VLSIPS™ technology as described in the above noted U.S. Patent No. 5,143,354 and PCT patent publication Nos. WO 90/15070 and 92/10092, is considered pionecring technology in the fields of combinatorial synthesis and screening of combinatorial libraries. More recently, patent Application Serial No. 08/082,937, filed June 25, 1993, describes methods for making arrays of oligonucleotide probes that can be used to provide a partial or complete sequence of a target nucleic acid and to detect the presence of a nucleic acid containing a specific oligonucleotide sequence.

### SUMMARY OF THE INVENTION

The present invention provides new compounds, compositions and methods which find application in solid phase synthesis including the preparation of high-density arrays of diverse polymer sequences such as diverse peptides and oligonucleotides as well as in preparation of arrays of diverse polymers. The compounds of the present invention are those which are typically referred to as linking groups, linkers or spacers.

According to the invention, novel compounds are provided which are unsymmetrical disulfide linking groups. These linking groups allow rapid and mild separation of the synthesized compound from the solid support. Such compounds have the formula; P¹-X¹-(W¹)ₙ-S-S-(W²)ₘ-X²-P². P¹ is a photolabile protecting group member selected from the group consisting of a hydrogen atom, an activating group and a protecting group. X¹ and X² are each independently selected from the group consisting of a bond, -O-, -NH-, -NR- and -CO₂-, wherein R is a lower alkyl group having one to four carbon atoms. W¹ and W² are each independently selected from the group consisting of methylene, oxyethylene and oxypropylene, n and m are each independently integers of from 2 to 12, n and m are not the same when W¹ and W² are the same. P¹ and P² are not both hydrogen atoms.

### BRIEF DESCRIPTION OF THE DRAWINGS

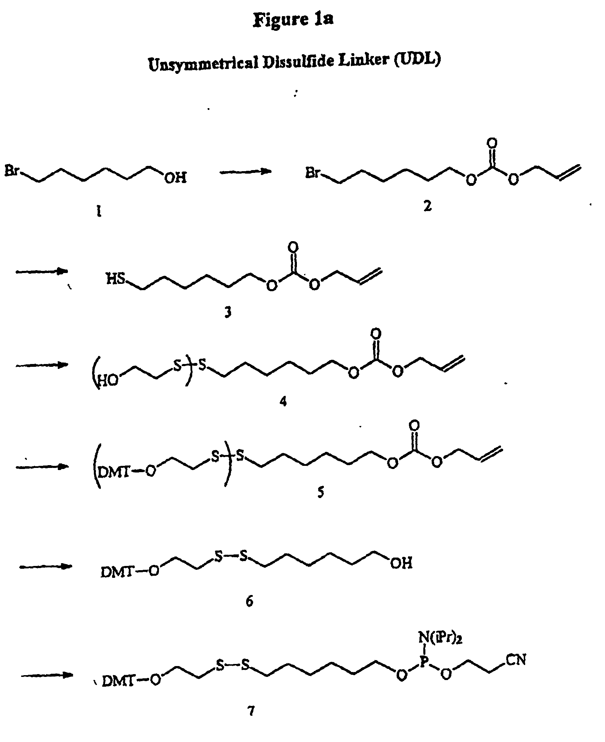

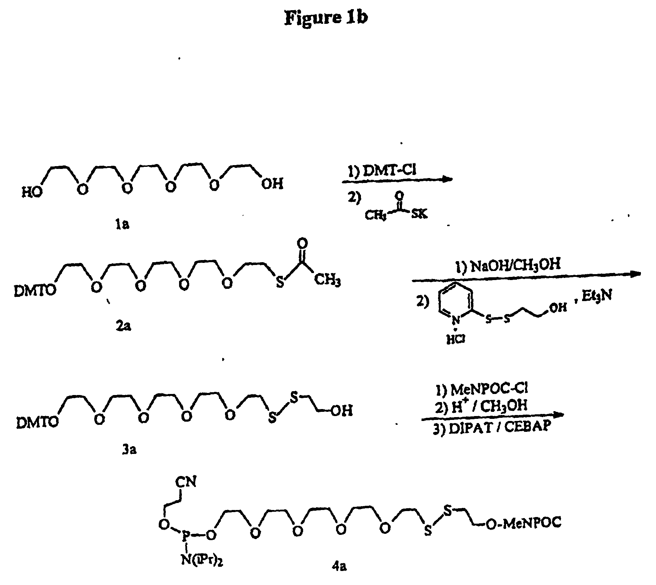

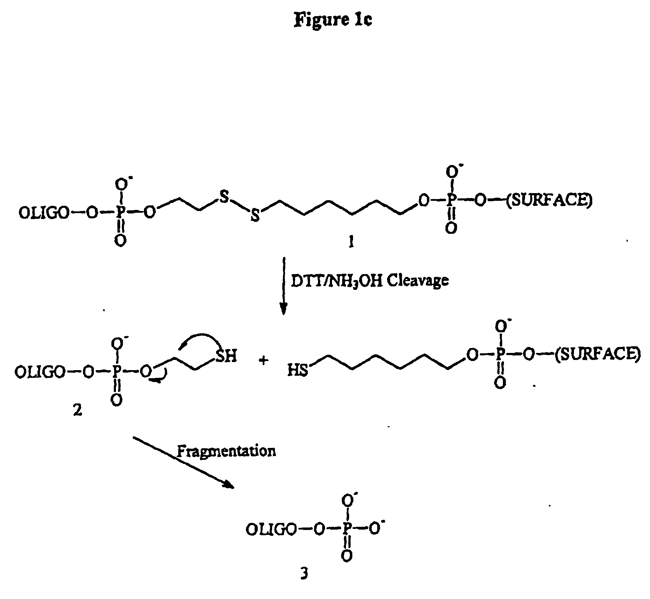

Figures 1A, 1B and 1C provide synthesis schemes for the preparation of unsymmetrical disulfide linkages.

### DETAILED DESCRIPTION OF THE INVENTION

### CONTENTS

I. Glossary
II. General
III. Novel Unsymmetrical Disulfide Linking Groups
IV. Examples
V. Conclusion

### I. Glossary

The following abbreviations are used herein: AcOH, acetic acid; ALLOC, allyloxycarbonyl; BOC, *t*-butyloxycarbonyl; BOP, benzotriazol-1-yloxy-tris(dimethylamino)phosphonium hexafluorophosphate; DIEA, diisopropylethylamine; DMF, dimethylformamide; DMT, dimethoxytrityl; DTT, dithiothreitol; EtOAc, ethyl acetate; FMOC, fluorenylmethyloxycarbonyl; MeNPOC, α-methylnitro-piperonyloxycarbonyl; MeNVOC, α-methylnitroveratryloxycarbonyl; mp, melting point; NVOC, nitroveratryloxycarbonyl; OBt, hydroxybenzotriazole radical; PBS, phosphate buffered saline; TFA, trifluoroacetic acid; DIPAT, diisopropylammonium tetrazolide; 2-CEBAP; 2-cyanoethyl tetraisopropylphosphorodiamidite; DDZ, α,α-dimethyl-3,5-dimethoxybenzyloxycarbonyl.

The following terms are intended to have the following general meanings as they are used herein:

Chemical terms: As used herein, the term "alkyl" refers to a saturated hydrocarbon radical which may be straight-chain or branched-chain (for example, ethyl, isopropyl, r-amyl, or 2,5-dimethylhexyl). When "alkyl" or "alkylene" is used to refer to a linking group or a spacer, it is taken to be a group having two available valences for covalent attachment, for example, -CH₂CH₂-, -CH₂CH₂CH₂-, -CH₂CH₂CH(CH₃)CH₂- and -CH₂(CH₂CH₂)₂CH₂-. Preferred alkyl groups as substituents are those containing 1 to 10 carbon atoms, with those containing 1 to 6 carbon atoms being particularly preferred. Preferred alkyl or alkylene groups as linking groups are those containing 1 to 20 carbon atoms, with those containing 3 to 6 carbon atoms being particularly preferred.

The term "aryl" as used herein, refers to an aromatic substituent which may be a single ring or multiple rings which are fused together, linked covalently or linked to a common group such as an ethylene or methylene moiety. The aromatic rings may each contain heteroatoms, for example, phenyl, naphthyl, biphenyl, diphenylmethyl, 2,2-diphenyl-1-ethyl, thienyl, pyridyl and quinoxalyl. The aryl moieties may also be optionally substituted with halogen atoms, or other groups such as nitro, carboxyl, alkoxy, phenoxy and the like. Additionally, the aryl radicals may be attached to other moieties at any position on the aryl radical which would otherwise be occupied by a hydrogen atom (such as, for example, 2-pyridyl, 3-pyridyl and 4-pyridyl). As used herein, the term "aralkyl" refers to an alkyl group bearing an aryl substituent (for example, benzyl, phenylethyl, 3-(4-nitrophenyl)propyl, and the like).

The term "protecting group" as used herein, refers to any of the groups which are designed to block one reactive site in a molecule while a chemical reaction is carried out at another reactive site. More particularly, the protecting groups used herein can be any of those groups described in Greene, *et al., Protective Groups In Organic Chemistry*, 2nd Ed., John Wiley & Sons, New York, NY, 1991. The proper selection of protecting groups for a particular synthesis will be governed by the overall methods employed in the synthesis. For example, in "light-directed" synthesis, discussed below, the protecting groups will be photolabile protecting groups such as dimethoxybenzoin, NVOC, MeNPOC, and those disclosed in WO 94/10128. In other methods, protecting groups may be removed by chemical methods and include groups such as FMOC, DMT and others known to those of skill in the an.

The term "activating agent" refers to those groups which, when attached to a particular functional group or reactive site, render that site more reactive toward covalent bond formation with a second functional group or reactive site. For example, the group of activating groups which are useful for a carboxylic acid include simple ester groups and anhydrides. The ester groups include alkyl, aryl and akenyl esters and in particular such groups as 4-nitrophenyl, N-hydroxylsuccinimide and pentafluorophenol. Other activating groups will include phosphodiester-forming groups such as phosphoramidates, phosphite-triesters, phosphotriesters, and H-phosphonates. Still other activating agents are known to those of skill in the art.

Monomer: A monomer is a member of the set of small molecules which are or can be joined together to form a polymer or a compound composed of two or more members. The set of monomers includes but is not restricted to, for example, the set of common L-amino acids, the set of D-amino acids, the set of synthetic and/or natural amino acids, the set of nucleotides and the set of pentoses and hexoses. The particular ordering of monomers within a polymer is referred to herein as the "sequence" of the polymer. As used herein, monomers refers to any member of a basis set for synthesis of a polymer. For example, dimers of the 20 naturally occurring L-amino acids form a basis set of 400 monomers for synthesis of polypeptides. Different basis sets of monomers may be used at successive steps in the synthesis of a polymer. Furthermore, each of the sets may include protected members which are modified after synthesis. The invention is described herein primarily with regard to the preparation of molecules containing sequences of monomers such as amino acids, but could readily be applied in the preparation of other polymers. Such polymers include, for example, both linear and cyclic polymers of nucleic acids, polysaccharides, phospholipids, and peptides having either α-, β-, or ω-amino acids, heteropolymers in which a known drug is covalently bound to any of the above, polynucleotides, polyurethanes, polyesters, polycarbonates, polyureas, polyamides, polyethyleneimines, polyarylene sulfides, polysiloxanes, polyimides, polyacetates, or other polymers which will be apparent upon review of this disclosure. Such polymers are "diverse" when polymers having different monomer sequences are formed at different predefined regions of a substrate. Methods of cyclization and polymer reversal of polymers are disclosed in copending application USSN 07/978940 which is a CIP of U.S. Patent No. 5,242,974 entitled "POLYMER REVERSAL ON SOLID SURFACES."

Polymer: A polymer is formed by covalent linkage of at least two monomer units. Polymers can incorporate any number of monomer units. Examples of polymers include oligonucleotides, peptides and carbohydrates.

Oligonucleotide: An oligonucleotide can be DNA or RNA (i.e, a nucleic acid), and single- or double-stranded. Oligonucleotides can be naturally occurring or synthetic. The segments are usually between 2 and 100 bases, but can be of any length. Lengths between 5-10, 5-20, 10-20, 10-50, 20-50 or 20-100 bases are common.

Peptide: A peptide is a polymer in which the monomers are amino acids and are joined together through amide bonds, alternatively referred to as a polypeptide. When the amino acids are α-amino acids, either the L-optical isomer or the D-optical isomer may be used. Additionally, unnatural amino acids, for example, β-alanine, phenylglycine and homoarginine are also meant to be included. Peptides are two or more amino acid monomers long, can be of any length and are often more than 20 amino acid monomers long.

Substrate: A material having a rigid or semi-rigid surface. In many embodiments, at least one surface of the substrate will be substantially flat, although in some embodiments it may be desirable to physically separate synthesis regions for different polymers with, for example, wells, raised regions, etched trenches, or the like. In some embodiments, the substrate itself contains wells, trenches, flow through regions, etc. which form all or part of the synthesis regions. According to other embodiments, small beads may be provided on the surface, and compounds synthesized thereon may be released upon completion of the synthesis.

A material having a plurality of grooves or recessed regions on a surface thereof. The grooves or recessed regions may take on a variety of geometric configurations, including but not limited to stripes, circles, serpentine paths, or the like. Channel blocks may be prepared in a variety of manners, including etching silicon blocks, molding or pressing polymers, etc.

Predefined Region: A predefined region is a localized area on a substrate which is, was, or is intended to be used for formation of a selected polymer and is otherwise referred to herein in the alternative as "reaction" region, a "selected" region, or simply a "region." The predefined region may have any convenient shape, e.g., circular, rectangular, elliptical, wedge-shaped, etc. In some embodiments, a predefined region and, therefore, the area upon which each distinct polymer sequence is synthesized is smaller than about 1 cm², more preferably less than 1 mm², and still more preferably less than 0.5 mm². In most preferred embodiments the regions have an area less than about 10,000 µm² or, more preferably, less than 100 µm². Within these regions, the polymer synthesized therein is preferably synthesized in a substantially pure form. Additionally, multiple copies of the polymer will typically be synthesized within any preselected region. The number of copies can be in the thousands to the millions.

### II. General

The compounds, substrates and methods of the present invention can be used in a number of solid phase synthesis applications, including light-directed methods, flow channel and sporting methods, pin-based methods and bead-based methods.

### Light-Directed Methods

"Light-directed" methods (which are one technique in a family of methods known as VLSIPS™ methods) are described in U.S. Patent No. 5,143,854. The light directed methods discussed in the '854 patent involve activating predefined regions of a substrate or solid support and then contacting the substrate with a preselected monomer solution. The predefined regions can be activated with a light source, typically shown through a mask (much in the manner of photolithography techniques used in integrated circuit fabrication). Other regions of the substrate remain inactive because they are blocked by the mask from illumination and remain chemically protected. Thus, a light pattern defines which regions of the substrate react with a given monomer. By repeatedly activating different sets of predefined regions and contacting different monomer solutions with the substrate, a diverse array of polymers is produced on the substrate. Of course, other steps such as washing unreacted monomer solution from the substrate can be used as necessary.

### Flow Channel or Spotting Methods

Additional methods applicable to library synthesis on a single substrate are described in U.S. Patent Nos.5,677,195 and 5,384,261. In the methods disclosed in these patents reagents are delivered to the substrate by either (1) flowing within a channel defined on predefined regions or (2) "spotting" on predefined regions. However, other approaches, as well as combinations of spotting and flowing, may be employed. In each instance, certain activated regions of the substrate are mechanically separated from other regions when the monomer solutions are delivered to the various reaction sites.

A typical "flow channel" method applied to the compounds and libraries of the present invention can generally be described as follows. Diverse polymer sequences are synthesized at selected regions of a substrate or solid support by forming flow channels on a surface of the substrate through which appropriate reagents flow or in which appropriate reagents are placed. For example, assume a monomer "A" is to be bound to the substrate in a first group of selected regions. If necessary, all or part of the surface of the substrate in all or a part of the selected regions is activated for binding by, for example, flowing appropriate reagents through all or some of the channels, or by washing the entire substrate with appropriate reagents. After placement of a channel block on the surface of the substrate, a reagent having the monomer A flows through or is placed in all or some of the channel(s). The channels provide fluid contact to the first selected regions, thereby binding the monomer A on the substrate directly or indirectly (via a spacer) in the first selected regions.

Thereafter, a monomer B is coupled to second selected regions, some of which may be included among the first selected regions. The second selected regions will be in fluid contact with a second flow channel(s) through translation, rotation, or replacement of the channel block on the surface of the substrate; through opening or closing a selected valve; or through deposition of a layer of chemical or photoresist. If necessary, a step is performed for activating at least the second regions. Thereafter, the monomer B is flowed through or placed in the second flow channel(s), binding monomer B at the second selected locations. In this particular example, the resulting sequences bound to the substrate at this stage of processing will be, for example, A, B, and AB. The process is repeated to form a vast array of sequences of desired length at known locations on the substrate.

After the substrate is activated, monomer A can be flowed through some of the channels, monomer B can be flowed through other channels, a monomer C can be flowed through still other channels, etc. In this manner, many or all of the reaction regions are reacted with a monomer before the channel block must be moved or the substrate must be washed and/or reactivated. By making use of many or all of the available reaction regions simultaneously, the number of washing and activation steps can be minimized.

One of skill in the art will recognize that there are alternative methods of forming channels or otherwise protecting a portion of the surface of the substrate. For example, according to some embodiments, a protective coating such as a hydrophilic or hydrophobic coating (depending upon the nature of the solvent) is utilized over portions of the substrate to be protected, sometimes in combination with materials that facilitate wetting by the reactant solution in other regions. In this manner, the flowing solutions are further prevented from passing outside of their designated flow paths.

The "spotting" methods of preparing compounds and libraries of the present invention can be implemented in much the same manner as the flow channel methods. For example, a monomer A can be delivered to and coupled with a first group of reaction regions which have been appropriately activated. Thereafter, a monomer B can be delivered to and reacted with a second group of activated reaction regions. Unlike the flow channel embodiments described above, reactants are delivered by directly depositing (rather than flowing) relatively small quantities of them in selected regions. In some steps, of course, the entire substrate surface can be sprayed or otherwise coated with a solution. In preferred embodiments, a dispenser moves from region to region, depositing only as much monomer as necessary at each stop. Typical dispensers include a micropipette to deliver the monomer solution to the substrate and a robotic system to control the position of the micropipette with respect to the substrate, or an ink-jet printer. In other embodiments, the dispenser includes a series of tubes, a manifold, an array of pipettes, or the like so that various reagents can be delivered to the reaction regions simultaneously.

### Pin-Based Methods

Another method which is useful for the preparation of compounds and libraries of the present invention involves "pin based synthesis." This method is described in detail in U.S. Patent No. 5,288,514. The method utilizes a substrate having a plurality of pins or other extensions. The pins are each inserted simultaneously into individual reagent containers in a tray. In a common embodiment, an array of 96 pins/containers is utilized.

Each tray is filled with a particular reagent for coupling in a particular chemical reaction on an individual pin. Accordingly, the trays will often contain different reagents. Since the chemistry disclosed herein has been established such that a relatively similar set of reaction conditions may be utilized to perform each of the reactions, it becomes possible to conduct multiple chemical coupling steps simultaneously. In the first step of the process the invention provides for the use of substrate(s) on which the chemical coupling steps are conducted. The substrate is optionally provided with a spacer having active sites. In the particular case of oligonucleotides, for example, the spacer may be selected from a wide variety of molecules which can be used in organic environments associated with synthesis as well as aqueous environments associated with binding studies. Examples of suitable spacers are polyethyleneglycols, dicarboxylic acids, polyamines and alkylenes, substituted with, for example, methoxy and etboxy groups. Additionally, the spacers will have an active site on the distal end. The active sites are optionally protected initially by protecting groups. Among a wide variety of protecting groups which are useful are FMOC, BOC, t-butyl esters, t-butyl ethers, and the like. Various exemplary protecting groups are described in, for example, Atherton *et al., Solid Phase Peptide Synthesis*, IRL Press (1989). In some embodiments, the spacer may provide for a cleavable function by way of, for example, exposure to acid or base.

### Bead Based Methods

Yet another method which is useful for synthesis of polymers and small ligand molecules on a solid support "bead based synthesis." A general approach for bead based synthesis is described in U.S. Patent. Nos. 5,770,358, 6,384,261 5,693,603 5,541,061 and WO 93/22684.

For the synthesis of molecules such as oligonucleotides on beads, a large plurality of beads are suspended in a suitable carrier (such as water) in a container. The beads are provided with optional spacer molecules having an active site. The active site is protected by an optional protecting group.

In a first step of the synthesis, the beads are divided for coupling into a plurality of containers. For the purposes of this brief description, the number of containers will be limited to three, and the monomers denoted as A, B, C, D, E, and F. The protecting groups are then removed and a first portion of the molecule to be synthesized is added to each of the three containers (*i.e*., A is added to container 1, B is added to container 2 and C is added to container 3).

Thereafter, the various beads are appropriately washed of excess reagents, and remixed in one container. Again, it will be recognized that by virtue of the large number of beads utilized at the outset, there will similarly be a large number of beads randomly dispersed in the container, each having a particular first portion of the monomer to be synthesized on a surface thereof.

Thereafter, the various beads are again divided for coupling in another group of three containers. The beads in the first container are deprotected and exposed to a second monomer (D), while the beads in the second and third containers are coupled to molecule portions E and F respectively. Accordingly, molecules AD, BD, and CD will be present in the first container, while AE, BE, and CE will be present in the second container, and molecules AF, BF, and CF will be present in the third container. Each bead, however, will have only a single type of molecule on its surface. Thus, all of the possible molecules formed from the first portions A, B, C, and the second portions D, E, and F have been formed.

The beads are then recombined into one container and additional steps such as are conducted to complete the synthesis of the polymer molecules. In a preferred embodiment, the beads are tagged with an identifying tag which is unique to the particular compound which is present on each bead. A complete description of identifier tags for use in synthetic libraries is provided in U.S. Patent No. 5,693,603.

### Utilities of Chemical Libraries

The advent of methods for the synthesis of diverse chemical compounds on solid supports has resulted in the genesis of a multitude of diagnostic applications for such chemical libraries. A number of these diagnostic applications involve contacting a sample with a solid support, or chip, having multiple attached biological polymers such as peptides and oligonucleotides, or other small ligand molecules synthesized from building blocks in a stepwise fashion, in order to identify any species which specifically binds to one or more of the attached polymers or small ligand molecules.

For example, patent Application Serial No. 08/082,937, filed June 25, 1993, describes methods for making arrays of oligonucleotide probes that can be used to provide the complete sequence of a target nucleic acid and to detect the presence of a nucleic acid containing a specific oligonucleotide sequence. U.S. Patent No. 5,556,752, describes methods of making arrays of unimolecular, double-stranded oligonucleotides which can be used in diagnostic applications involving protein/DNA binding interactions such as those associated with the p53 protein and the genes contributing to a number of cancer conditions. Arrays of double-stranded oligonucleotides can also be used to screen for new drugs having particular binding affinities. The linking groups and labels provided herein are useful in each of these library applications, as well as others now known in the literature.

### III. Novel Unsymmetrical Disulfide Linking Groups

The present invention provides novel unsymmetrical disulfide linking groups which can facilitate oligomer or small molecule synthesis on a solid support and which can provide rapid release from the support under very mild conditions.

One group of unsymmetrical disulfide compounds which can be used as linking groups are represented by the formula:

P¹-X¹-(W¹)ₙ-S-S-(W²)ₘ-X²-P² (I)

In this formula, P¹ is a photolabile protecting group and P² a hydrogen atom, an activating group (*e.g*., a phosphodiester-forming group) or a selectively removable protecting group. The symbols X¹ and X² each independently represent a bond, -O-, -NH-, -NR- and -CO₂-, in which R is an alkyl group having one to four carbon atoms. The symbols W¹ and W² each independently represent a methylene group (-CH₂-), an oxyethylene group (-OCH₂CH₂- or -CH₂CH₂O-), an oxypropylone group (*e.g*., -OCH₂CH₂CH₂-, -OCH₂CH(CH₃)- or -CH(CH₃)CH₂O-). The letters n and m each independently represent integers of from 2 to 12, with the proviso that n and m are not the same integer when W¹ and W² are identical. Preferably, the letters n and m represent integers of from 3 to 8. All numerical ranges in this application are meant to be inclusive of their upper and lower limits.

P¹ is a photocleavable protecting group, preferably an NVOC, MeNPOC, Dimethoxybenzoinyl, or α,α-dimethyl-3,5-dimethoxybenzyloxy carbonyl (DDZ). More preferably, P¹ is a MeNPOC protecting group.

The linking groups of formula (I) are useful as a 3'-end cleavable linking group in any solid phase synthesis of oligonucleotides. When used for this solid phase preparation of oligonucleotides, P² is preferably an activating group such as a phosphoramidite or other functionally equivalent group commonly used in solid phase oligonucleotide synthesis. Detailed descriptions of the procedures for solid phase synthesis of oligonucleotides by phosphite-triester, phosphotriester, and H-phosphonate chemistries are widely available. See, for example, Itakura, U.S. Pat. No. 4,401,796; Caruthers, *et al*., U.S. Pat. Nos. 4,458,066 and 4,500,707; Beaucage, *et al*., *Tetrahedron Lett*., 22:1859-1862 (1981); Matteucci, *et al*., *J. Am. Chem. Soc*., **103**:3185-3191 (1981); Caruthers, *et al., Generic Engineering,* **4**:1-17 (1982); Jones, chapter 2, Atkinson, *et al*., chapter 3, and Sproat, *et al.,* chapter 4, in *Oligonucleotide Synthesis: A Practical Approach,* Galt (ed.), IRL Press, Washington D.C. (1984); Froehler, *et al*., *Tetrahedron Lett*., 27:469-472 (1986); Froehler, *et al*., *Nucleic Acids Res*., 14:5399-5407 (1986); Sinha, *et al. Tetrahedron Lett*., **24**:5843-5846 (1983); and Sinha, *et al., Nucl. Acids Res*., **12**:4539-4557 (1984). In these embodiments X² is preferably -O-.

The unsymmetrical disulfide linking groups of the present invention can be prepared by methods which are known to those of skill in the art. Figures 1a and 1b provide synthesis schemes for preparation of the linkers. According to Figure 1a, commercially available 6-bromohexanol (Aldrich Chemical Company, Milwaukee, Wisconsin, USA) can be treated with allyl chloroformate in pyridine to produce the allyl carbonate (2 in Figure 1a). The terminal bromide can be converted to a thiol upon treatment with sodium hydrogen sulfide in THF/H₂O at pH 7. Disulfide functionality can then be introduced upon reaction of thiol 4 with 2-pyridyl-2-hydroxyethyl disulfide in THF and triethylamine. Protection of the terminal hydroxyl group is accomplished with DMT chloride in pyridine to provide **6.** Conversion of 6 to 8 can be achieved by removal of the allyl carbonate (catalytic K₂CO₃ in MeOH) and treatment of the resultant hydroxyl group with DiPAT and BAP. As can be seen, this methodology provides a linking group of the formula above in which P¹ is DMT, X¹ is -O-, n is 2, m is 6, X² is -O- and P² is phosphoramidite.

As shown in Fig. 1C, when N=2, the disulfide bond of 1 cleaves under netural or basic conditions in the presence of DTT to give an oligonucleotide **2**, which possess the 2-mercaptoehtyl phosphate ester at the 3'-end. This ester fragments have been observed efficiently to produce the 3-phosphorylated oligonucleotide **3**. This product has been shown to be identical to that produced by the base catalyzed cleavage of oligonucleotides tethered to the surface via the known Phosphate-ON reagent (Glen Research). The unsymmetrical disulfie linker when N=2 is preferred when it is desirable to cleave from the surface and analyze the oligonucleotides by HPLC, since the resultant oligonucleotides do not posses a 3'-thiol appendage. In the cases where N>2, the mercaptoalkyl esters should be more stable and the cleaved oligonucleotides retain the corresponding thiol appendage. This makes subsequent analysis of the cleaved DNA difficult because of oxidation of the thiol group.

The steps just described can be suitably modified by one of skill in the art to prepare a number of related analogs based upon the availability of brome alcohols (1 as starting material) and 2-pyridyl disulfide alcohols. According to Figure 1b, pentaethylene glycol (**1a**) is first protected with DMT-Cl, then converted to a mono thioester (**2a**) with potassium thioacetate. Cleavage of the acetyl group with base, and reaction of the resultant thiol functionality with 2-pyridyl 3-hydroxyethyl disulfide provides a mono-protected unsymmetrical disulfide linker (3a). Protection of the hydroxyl group with MeNPOC-Cl, followed by removal of the DMT protecting group and conversion of the liberated hydroxyl group to a phosphoramidite, provides linker (4a) which is useful in automated oligomer synthesizers.

In a related aspect, the present invention provides modified substrates which are useful in the solid phase synthesis of oligonucleotides as well as arrays of diverse polymers. The substrates are derivatized with the unsymmetrical disulfide linking groups described above and are represented by the formula:

A¹-B¹-L¹ (II)

in which A¹ is a solid substrate, B¹ is a bond or a spacer and L¹ is an unsymmetrical disulfide linking group having the formula:

P¹-X¹-(W¹)ₙ-S-S-(W¹)ₘ-X²- (IIa)

In formula (IIa), the symbol P¹ represents a protecting photolabile group. The symbols X¹ and X² are each independently a bond, -O-, -NH-, -NR- and -CO₂-, wherein R is a lower alkyl group having one to four carbon atoms. The symbols W¹ and W² are as described above. The letters n and m represent, as above, integers of from 2 to 12 with the understanding that n and m are not the same when W¹ and W² are identical,

In this aspect of the invention, the solid substrates may be biological, nonbiological, organic, inorganic, or a combination of any of these, existing as particles, strands, precipitates gels, sheets, tubing, spheres, containers, capillaries, pads, slices, films, plates, slides, *etc*. The solid substrate is preferably flat but may take on alternative surface configurations. For example, the solid substrate may contain raised or depressed regions on which synthesis takes place. In some embodiments, the solid substrate will be chosen to provide appropriate light-absorbing characteristics. For example, the substrate may be a polymerized Langmuir Blodgett film, functionalized glass, Si, Ge, GaAs, GaP, SiO₂, SiN₄, modified silicon, or any one of a variety of gels or polymers such as (poly)tetrafluoroethylene, (poly)vinylidendifluoride, polystyrene, polycarbonate, or combinations thereof. Other suitable solid substrate materials will be readily apparent to those of skill in the art. Preferably, the surface of the solid substrate will contain reactive groups, which are carboxyl, amino, hydroxyl, thiol, or the like. More preferably, the surface will be optically transparent and will have surface Si-OH functionalities, such as are found on silica surfaces.

For those embodiments in which B¹ is a spacer, it will be attached to the solid substrate via carbon-carbon bonds using, for example, substrates having (poly)trifluorochloroethylene surfaces, or more preferably, by siloxane bonds (using, for example, glass or silicon oxide as the solid substrate). Siloxane bonds with the surface of the substrate are formed in one embodiment via reactions of derivatization reagents bearing trichlorosilyl or trialkoxysilyl groups.

The particular spacer can be selected based upon its hydrophilic or hydrophobic properties to improve presentation of an attached oligomer or compound to certain receptors, proteins or drugs. Prior to attachment to the solid substrate the spacer will have a substrate attaching group at one end, and a reactive site at the other end. The reactive site will be a group which is appropriate for attachment to the linking group, L¹. For example, groups appropriate for attachment to a silica surface would include trichlorosilyl and trialkoxysilyl functional groups. Groups which are suitable for attachment to a linking group include amine, hydroxyl, thiol, carboxylic acid, ester, amide, isocyanate and isothiocyanate. Preferred spacers include aminoalkyltrialkoxysilanes, hydroxyalkyltrialkoxysilanes, polyethyleneglycois, polyethyleneimine, polyacrylamide, polyvinylalcohol and combinations thereof.

The unsymmetrical disulfide linking groups used in the present modified substrates are represented by radicals of the formula:

P¹-X¹-(W¹)ₙ-S-S-(W²)ₘ-X²- (IIa)

in which X¹, W¹, W², n, m, X² and P¹ have the meanings as provided above. In preferred embodiments, n and m are integers of from 3 to 8, and W¹ and W² are either methylene groups or oxyethylene groups. In other preferred embodiments, X¹ and X² are each independently -O- or -NH-, most preferably X¹ and X² are both -O-. Im yet other preferred embodiments W¹ and W² are both methylene, and X¹ and X² are both -O- ; or X¹ and X² are both -O-, and n and m are each integers of from 2 to 8. In still other preferred embodiments, P¹ is a DMT group.

Attachment of the linking group L¹ to a functional group on the solid support or to a reactive site on a spacer can be accomplished using standard chemical methods. For example, when X² is oxygen, linkage to a carboxylic acid or activated carboxylic acid can be made using standard ester-forming reactions. Alternatively, the X² group (derived from a hydroxyl group) can be reacted with support bound isocyanate groups to form carbamate linkages. In other embodiments, X² will be attached to a solid support via a phosphodiester or phosphotriester linkage. In these embodiments, the attachment will typically occur via a phosphoamidite or other phosphodiester or triester-forming group initially present on the unsymmetrical disulfide linking group.

In jet another aspect, the present invention provides methods for the preparation of an array of diverce polymers on a solid support. The methods typically comprise:
(a) contacting a solid support with an unsymmetrical disulfide linking group of formula:

   P¹-X¹-(W¹)ₙ-S-S-(W²)ₘ-X²-P² (IIb)

   in which P¹ and P² are each members independently selected from the group consisting of a hydrogen atom, an activating group and a protecting group; X¹ and X² are each independently a bond, -O-, -NH-, -NR- or -CO-, wherein R is a lower alkyl group having one to four carbon atoms; the symbols W¹ and W² each independently represent a methylene group (-CH₂-), an oxyethylene group (-OCH₂CH₂- or -CH₂CH₂O-), an oxypropylene group (*e.g*., -OCH₂CH₂CH₂-, -OCH₂CH(CH₃)- or -CH(CH₃)CH₂O-), and the like; and n and m are each independently integers of from 2 to 12 with the proviso that n and m are not the same when W¹ and W² are the same, under conditions sufficient to produce a derivatized solid support having attached unsymmetrical disulfide linking groups suitably protected with protecting groups;
(b) optionally removing the protecting groups from the derivatized solid support to provide a derivatized solid support having unsymmetrical disulfide linking groups with synthesis initiation sites; and
(c) coupling diverse polymers to the synthesis initiation sites on the derivatized solid support to produce a solid support having attached an array of diverse polymers which are removable therefrom upon application of a disulfide cleaving reagent.

Attaching the unsymmetrical disulfide linking group radical to the solid support can generally be carried out by standard chemical methods such as those described above. In a preferred embodiment, X² is -O- and P² is a phosphoramidite. In this embodiment the unsymmetrical disulfide linking group can be reacted with a solid support having available hydroxyl groups using standard nucleic acid synthesis techniques. The product is a solid support having unsymmetrical disulfide linking groups which are attached via a. phosphodiester linkage. The distal end of the linking group (that end furthest removed from the solid support will be either a synthesis initiation site or a protected synthesis initiation site.

When present, the optional protecting groups can be removed using well known methods which will not interfere with molecules or groups present on the support. In preferred embodiments, the removal of protecting groups can be carried out at particular predefined regions on the support using light and photolithographic masks, or flow channel or spotting techniques with appropriate removal reagents. The removal of the protecting groups provides a solid support having attached unsymmetrical disulfide linkages and synthesis initiation sites.

The preparative methods then continue with the coupling of monomers, molecules or components of molecules to the synthesis initiation sites. Again, the chemistry of coupling follows standard synthesis methodology known to those of skill in the art.

Preferred embodiments for this aspect of the invention are generally as described above for the related compounds and modified substrates. In a particularly preferred embodiment, -(W¹)ₙ- is -CH₂CH₂-.

### IV. EXAMPLES

### EXAMPLE 1

This example illustrates the synthesis of an unsymmetrical disulfide linking group having a DMT protecting group at one terminus and a phosphoramidite activating group at the other terminus.

### (a) Conversion of 6-bromohexanol to 6-bromohexyl allyl carbonate

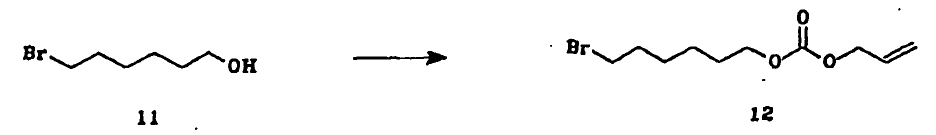

To a solution of 6-bromobexanol (4.8 g) in 20 mL of pyridine at 0°C to 20°C, was added allyl chloroformate (4.5 mL, 40 mmol). The resulting mixture was stirred at room temperature overnight. Diethyl ether was added and the mixture was washed sequentially with saturated NaHCO₃, water, and saturated NaCl. The organic layer was dried over anhydrous Na₂SO₄, filtered and evaporated under reduced pressure to provide crude 6-bromohexyl allyl carbonate 12 (3.627 g) as a colorless oil. Flash chromatography (hexane/Et₂O, 2/1 as eluant) provided the purified product 12 (2.158 g).

### (b) Preparation of 6-mercaptohexyl allyl carbonate

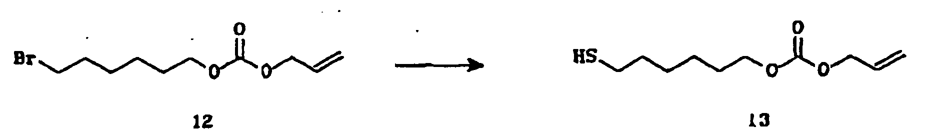

Sodium hydrogen sulfide (3.6 g) was dissolved in aqueous pH 7 buffer (10 mL) and 6-bromohexyl allyl carbonate (0.4 g) was added. Tetrahydrofuran (~15 mL) was added and the mixture was stirred at room temperature overnight. The mixture was diluted with 25 mL of diethylether. The organic layer was washed with water and brine and dried over anhydrous Na₂SO₄. The salt was removed by filtration and the solvent was removed from the filtrate to provide a crude product which was used in the next step without further purification.

### (c) Preparation of disulfide 14

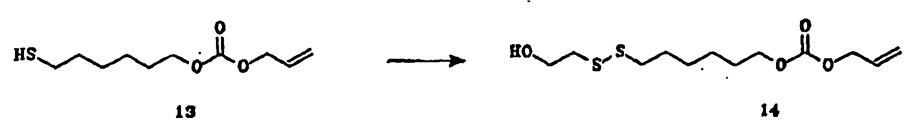

6-Mercaptohexyl allyl carbonate 13 (388 mg), 2-hydroxyethyl 2-pyridyl disulfide (350 mg) and triethylamine (1.0 mL) were combined in 5 mL of THF. The reaction was stirred for 1 hr. The mixture was concentrated under reduced pressure and the residue was purified by flash chromatography (silica; hexane/diethyl ether, 1/1) to provide the desired disulfide 14 (422 mg) as a clear colorless oil.

### (d) Preparation of Monoprotected Disulfide 16

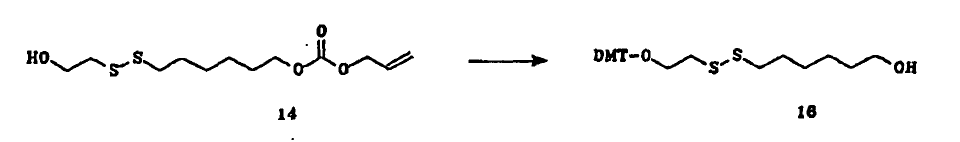

The hydroxy disulfide 14 (1.8 g, 6.1 mmol, 1.0 eq.) was combined with DMT-Cl (2.4 g, 6.7 mmol, 1.1 eq.) in 20 mL of dry pyridine under an atmosphere of argon. After 4 hr the mixture was diluted with ethyl acetate (50 mL) and poured into 50 mL of saturated aqueous NaHCO₃. The resulting mixture was extracted with EtOAc (50 mL), and the organic extract was washed with saturated aqueous NaCl and dried over anhydrous Na₂SO₄. Evaporation of the solvent provided the crude intermediate product 16 as an orange oil.

The oil was combined with 50 mL of 20% THF in anhydrous MeOH and a catalytic amount of anhydrous K₂CO₃ was added. The mixture was stirred at room temperature overnight.. EtOAc (50 mL) was added and the resulting mixture was poured into 50 mL of saturated aqueous NaHCO₃. The layers were separated and the aqueous portion was extracted with two 50 mL portions of ethyl acetate. The combined organic portions were washed with saturated aqueous NaCl and dried over anhydrous Na₂O₄. Evaporation of the solvent provided the crude product as an orange oil. Purification was carried out by flash chromatography (hexane/EtOAc, 3/7 with 1% triethylamine) to provide 2.65 g (73 % for the two steps) of product 16 as a pale yellow oil.

### (e) Preparation of DMT-protected, Phosphoramidite (UDL) 17

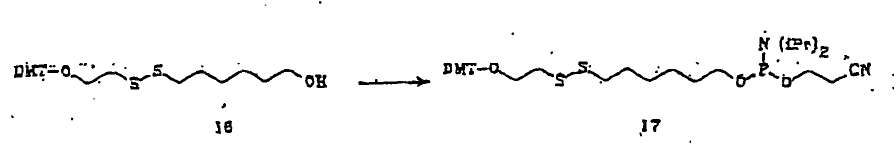

DMT-protected disulfide 16 (2.0 g, 3.35 mmol, 1.0 eq.) and DIPAT (2.87 g, 1.68 mmol 0.5 eq.) were combined in 20 mL of dry CH₂Cl₂ under an atmosphere of argon. CEHAP (1.1 g, 1.2 mL, 3.69 mmol, 1.1 eq.) was added and the mixture was stirred at room temperature for 3 hr. The resulting mixture was poured into saturated aqueous N₂HCO₃ and the organic layer was separated, washed with saturated aqueous NaCl and dried over Na₂SO₄. Removal of solvent under reduced pressure provided the DMT-protected, phosphoramidite 17 as a pale yellow oil. Purification by flash chromatography, cluting first with hexane containing 1% triethylamine, then with ethyl acetate/hexane (1/9, containing 1% triethylamine), provided 2.06 g (77%) of 17 as a pale yellow oil. ¹H NMR and ³¹P NMR were consistent with the assigned structure.

The unsymmetrical disulfide linking group can be used in any automated synthesizer under conditions which are useful for standard phosphoramidites. Typically, the molarity of the iodine solution used in these syntheses is reduced from 0.2 M to 0.02 M.

## Claims

1. A compound having the formula:
P¹X¹-(W¹)ₙ-S-S-(W²)ₘ-X²P² (I)
wherein
P¹ is a photolabile protecting group and P² a member selected from the group consisting of a hydrogen atom, an activating group and a protecting group;
X¹ and X² are each independently selected from the group consisting of a bond, -O-, -NH-, -NR- and -CO₂-, wherein R is a lower alkyl group having one to four carbon atoms;
W¹ and W² are each independently selected from the group consisting of methylene, oxyethylene and oxypropylene; and
n and m are each independently integers of from 2 to 12 with the proviso that n and m are not the same when W¹ and W² are the same, and with the further proviso that P¹ and P² are not both hydrogen atoms.

2. A modified substrate for use in solid phase chemical synthesis, said substrate having the formula:
A¹-B¹-L¹ (II)
wherein A¹ is a solid support, B¹ is a bond or a derivatizing group, and L¹ is a linking group having the formula:
P¹-X¹-(W¹)ₙ-S-S-(W²)ₘ-X²- (IIa)
wherein,
P¹ is a photolabile protecting group;
X¹ and X² are each independently selected from the group consisting of a bond, -O-, -NH-, -NR- and -CO₂-, where-in R is a lower alkyl group having one to four carbon atoms;
W¹ and W² are each independently selected from the group consisting of methylene, oxyethylene and oxypropylene; and
n and m are each independently integers of from 2 to 12 with the proviso that n and m are not the same when W¹ and W² are the same.

3. A substrate in accordance with claim 2, wherein W¹ and W² are both methylene, and X¹ and X² are both -O-.

4. A substrate in accordance with claim 2, wherein X¹ and X² are both -O-, and n and m are each integers of from 2 to 8.

5. A method of synthesizing an array of diverse polymers on a solid support having optional spacers, said polymers being removable therefrom upon treatment with a suitable disulfide cleaving reagent, said method comprising:
(a) contacting a solid support with an unsymmetrical disulfide linking group of formula:
P¹-X¹-(W¹)ₙ-S-S-(W²)ₘ-X²-P² (IIb)
wherein,
P¹ and P² are each members independently selected from the group consisting of a hydrogen atom, an activating group and a protecting group;
X¹ and X² are each independently selected from the group consisting of a bond, -O-, -NH-, -NR- and -CO₂-, wherein R is a lower alkyl group having one to four carbon atoms;
W¹ and W² are each independently selected from the group consisting of methylene, oxyethylene and oxypropylene; and
n and m are each independently integers of from 2 to 12 with the proviso that n and m are not the same when W¹ and W² are the same;
to produce a derivatized solid support having attached unsymmetrical disulfide linking groups suitable protected with protecting groups;
(b) optionally removing said protecting groups from said derivatized solid support to provide a derivatized solid support having unsymmetrical disulfide linking groups with synthesis initiation sites; and
(c) coupling said polymers to said synthesis initiation sites on said derivatized solid support to produce a solid support having attached an array of diverse polymers which are removable therefrom upon application of said disulfide cleaving reagent.

6. A method of synthesizing an array of diverse polymers on a substrate, comprising:
(a) providing a modified subtrate for use in solid phase chemical synthesis, said substrate having the formula:
A¹-B¹-L¹ (II)
wherein A is a solid support, B is a bond or a spacer group, and L is a linking group having the formula:
P¹-X¹-(W¹)ₙ-S-S-(W²)ₘ-X²- (Iia)
wherein:
P¹ is a protecting group;
X¹ and X² are each independently selected from the group consisting of a bond, -O-, -NH-, -NR- and -CO₂-, wherein R is a lower alkyl group having one to four carbon atoms;
W¹ and W² are each independently selected from the group consisting of methylene, oxyethylene and oxypropylene; and
n and m are each independently integers of from 2 to 12 with the proviso that n and m are not the same when W¹ and W² are the same; and
(b) preparing an array of diverse polymers on said modified substrate.

7. The method of claim 6, wherein P¹ is a photolabile protecting group.

8. The method of claim 6, wherein P¹ is a photolabile protecting group, W¹ and W² are both methylene, and X¹ and X² are both -O-.

9. The method of claim 6, wherein P¹ is a photolabile protecting group, X¹ and X² are both -O-, and n and m are each integers of from 2 to 8.

10. The method of claim 6, wherein P¹ is DMT, X¹ and X² are both -O-, W¹ and W² are both methylene, and n and m are each integers of from 2 to 8.

11. The methods of claims 9 or 10, wherein n is 2.

12. The method of claim 6, wherein the polymers are peptides.

13. The method of claim 6, wherein the polymers are polynucleotides.

14. The method of claim 6, wherein the preparing an array comprises:
(i) optionally removing said protecting groups from said modified substrate to provide a modified support with synthesis initiation sites; and
(ii) coupling monomers to said synthesis initiation sites on said modified substrate to produce a modified substrate having an array of diverse polymers.

15. The method of claim 14, wherein the selected regions of the modified support are activated with light.

16. The method of claim 6, further comprising releasing the polymers from the modified support.

## Patentansprüche

1. Verbindung der Formel
P¹X¹-(W¹)ₙ-S-S-(W²)ₘ-X²P² (I),
worin
P¹ eine lichtempfindliche Schutzgruppe und P² ein Mitglied ausgewählt aus der Gruppe bestehend aus einem Wasserstoffatom, einer aktivierenden Gruppe und einer Schutzgruppe ist;
X¹ und X² jeweils unabhängig voneinander aus der Gruppe bestehend aus einer Bindung, -O-, -NH-, -NR- und -CO₂-ausgewählt sind, wobei R eine niedere Alkylgruppe mit ein bis vier Kohlenstoffatomen ist;
W¹ und W² jeweils unabhängig voneinander aus der Gruppe bestehend aus Methylen, Oxyethylen und Oxpropylen ausgewählt sind; und
n und m jeweils unabhängig voneinander ganze Zahlen von 2 bis 12 sind, mit der Bedingung, daß n und m nicht gleich sind, wenn W¹ und W² gleich sind, und mit der weiteren Bedingung, daß P¹ und P² nicht beide Wasserstoffatome sind.

2. Modifiziertes Substrat zur Verwendung in der chemischen Festphasensynthese, wobei das Substrat die folgende Formel aufweist:
A¹-B¹-L¹ (II),
worin A¹ ein fester Träger ist, B¹ eine Bindung oder eine derivatisierende Gruppe ist, und L¹ eine Verknüpfungsgruppe der Formel:
P¹-X¹-(W¹)ₙ-S-S-(W²)ₘ-X²- (IIa),
ist worin
P¹ eine lichtempfindliche Schutzgruppe ist;
X¹ und X² jeweils unabhängig voneinander aus der Gruppe bestehend aus einer Bindung, -O-, -NH-, -NR- und -CO₂-ausgewählt sind, wobei R eine niedere Alkylgruppe mit ein bis vier Kohlenstoffatomen ist;
W¹ und W² jeweils unabhängig voneinander aus der Gruppe bestehend aus Methylen, Oxyethylen und Oxypropylen ausgewählt sind; und
n und m jeweils unabhängig voneinander ganze Zahlen von 2 bis 12 sind, mit der Bedingung, daß n und m nicht gleich sind, wenn W¹ und W² gleich sind.

3. Substrat nach Anspruch 2, bei dem W¹ und W² beide Methylen sind, und bei dem X¹ und X² beide -O- sind.

4. Substrat nach Anspruch 2, bei dem X¹ und X² beide -O-sind, und bei dem n und m jeweils ganze Zahlen von 2 bis 8 sind.

5. Verfahren zur Synthese einer Anordnung verschieder Polymeren auf einem festen Träger, der optional Abstandshalter aufweist, wobei die Polymere von diesem durch Behandlung mit einem geeignetem Disulfit-spaltenden Reagenz lösbar sind, wobei das Verfahren Schritte umfasst, bei denen man:
(a) einen festen Träger mit einer asymmetrischen Disulfit-Verknüpfungsgruppe der Formel:
P¹-X¹-(W¹)ₙ-S-S-(W²)ₘ-X²-P² (IIb),
kontaktiert, worin
P¹ und P² jeweils unabhängig voneinander ausgewählte Mitglieder der Gruppe bestehend aus einem Wasserstoffatom, einer aktivierenden Gruppe und einer Schutzgruppe sind;
X¹ und X² jeweils unabhängig voneinander aus der Gruppe bestehend aus einer Bindung, -O-, -NH-, - NR- und -CO₂- ausgewählt sind, wobei R eine niedere Alkygruppe mit ein bis vier Kohlenstoffatomen ist;
W¹ und W² jeweils unabhängig voneinander aus der Gruppe aus Methylen, Oxyethylen und Oxypropylen ausgewählt sind; und
n und m jeweils unabhängig voneinander ganze Zahlen von 2 bis 12 sind, mit der Bedingung, daß n und m nicht gleich sind, wenn W¹ und W² gleich sind;
um einen derivatisierten festen Träger herzustellen, an den asymmetrische Disulfit-Verknüpfungsgruppen angeheftet sind, die in geeigneter Weise durch Schutzgruppen geschützt sind;
(b) die Schutzgruppen der derivatisierten festen Träger gegebenenfalls entfernt, um einen derivatisierten festen Träger bereitzustellen, der asymmetrische Disulfit-Verknüpfungsgruppen mit Synthesestartorten aufweist; und
(c) die Polymere an die Synthesestartorte auf dem derivatisierten festen Träger koppelt, um einen festen Träger herzustellen, an den eine Anordnung verschiedener Polymere geheftet ist, die von diesem durch Verwendung des Disulfit-spaltenden Reagenz lösbar sind.

6. Verfahren zur Synthese einer Anordnung verschiedener Polymeren auf einem Substrat, das Schritte umfasst bei denen man:
(a) ein modifiziertes Substrates zur Verwendung in der chemischen Festphasensynthese bereit stellt, wobei das Substrat die Formel aufweist:
A¹-B¹-L¹ (II),
worin
A ein fester Träger, B eine Bindung oder eine Abstandshaltergruppe ist, und L eine Verknüpfungsgruppe ist mit der Formel:
P¹-X¹-(W¹)ₙ-S-S-(W²)ₘ-X²- (IIa),
worin
P¹ eine Schutzgruppe ist;
X¹ und X² jeweils unabhängig voneinander aus der Gruppe bestehend aus einer Bindung, -O-, -NH-, - NR- und -CO₂- ausgewählt sind, wobei R eine niedere Alkylgruppe mit ein bis vier Kohlenstoffatomen ist;
W¹ und W² jeweils unabhängig voneinander aus der Gruppe bestehend aus Methylen, Oxyethylen und Oxypropylen ausgewählt sind;
n und m jeweils unabhängig voneinander ganze Zahlen von 2 bis 12 sind, mit der Bedingung, daß n und m nicht gleich sind, wenn W¹ und W² gleich sind; und
(b) eine Anordnung verschiedener Polymere auf dem modifizierten Substrat erstellt.

7. Verfahren nach Anspruch 6, bei dem P¹ eine lichtempfindliche Schutzgruppe ist.

8. Verfahren nach Anspruch 6, bei dem P¹ eine lichtempfindliche Schutzgruppe ist, W¹ und W² beide Methylen sind, und X¹ und X² beide -O- sind.

9. Verfahren nach Anspruch 6, bei dem P¹ eine lichtempfindliche Schutzgruppe ist, X¹ und X² beide -O- sind, und n und m jeweils ganze Zahlen von 2 bis 8 sind.

10. Verfahren nach Anspruch 6, bei dem P¹ DMT ist, X¹ und X² beide -O- sind, W¹ und W² beide Methylen sind, und n und m jeweils ganze Zahlen von 2 bis 8 sind.

11. Verfahren nach Anspruch 9 oder 10, bei dem n 2 ist.

12. Verfahren nach Anspruch 6, bei dem die Polymere Peptide sind.

13. Verfahren nach Anspruch 6, bei dem die Polymere Polynukleotide sind.

14. Verfahren nach Anspruch 6, bei dem das Erstellen einer Anordnung Schritte umfasst, bei denen man:
(i) die Schutzgruppen des modifizierten Substrats gegebenenfalls entfernt, um ein modifiziertes Substrat mit Synthesestartorten bereitzustellen; und
(ii) Monomere an die Synthesestartorte auf dem modifizierten Substrat koppelt, um ein modifiziertes Substrat mit einer Anordnung von verschiedenen Polymeren herzustellen.

15. Verfahren nach Anspruch 14, bei dem die ausgewählten Bereiche des modifizierten Trägers mit Licht aktiviert werden.

16. Verfahren nach Anspruch 6, dass ferner die Freisetzung der Polymere von dem modifizierten Träger umfasst.

## Revendications

1. Composé ayant la formule :
P¹X¹-(W¹)ₙ-S-S-(W²)ₘ-X²P² (I)
dans laquelle
P¹ est un groupe de protection photolabile et P² un membre choisi dans le groupe constitué par un atome d'hydrogène, un groupe activateur et un groupe protecteur ;
X¹ et X² sont choisis chacun indépendamment dans le groupe constitué par une liaison, -O-, -NH-, -NR- et -CO₂-, dans laquelle R est un groupe alkyle inférieur ayant de un à quatre atomes de carbone ;
W¹ et W² sont choisis chacun indépendamment dans le groupe constitué par le méthylène, l'oxyéthylène et l'oxypropylène ; et
n et m sont chacun indépendamment des nombres entiers de 2 à 12, à la condition que n et m ne soient pas les mêmes quand W¹ et W² sont les mêmes, et à l'autre condition que P¹ et P² ne soient pas tous les deux des atomes d'hydrogène.

2. Substrat modifié pour une utilisation dans la synthèse chimique en phase solide, ledit substrat ayant la formule :
A¹-B¹-L¹ (II)
dans laquelle A¹ est un support solide, B¹ est une liaison ou un groupe de dérivation, et L¹ est un groupe de liaison ayant la formule :
P¹-X¹-(W¹)ₙ-S-S-(W²)ₘ-X²- (IIa)
dans laquelle,
P¹ est un groupe protecteur photolabile ;
X¹ et X² sont choisis chacun indépendamment dans le groupe constitué par une liaison, -O-, -NH-, -NR- et -CO₂-, dans laquelle R est un groupe alkyle inférieur ayant de un à quatre atomes de carbone ;
W¹ et W² sont choisis chacun indépendamment dans le groupe constitué par le méthylène, l'oxyéthylène et l'oxypropylène ; et
n et m sont chacun indépendamment des nombres entiers de 2 à 12 à la condition que n et m ne soient pas les mêmes quand W¹ et W² sont les mêmes.

3. Substrat selon la revendication 2, dans lequel W¹ et W² sont tous les deux un méthylène, et X¹ et X² sont tous les deux -O-.

4. Substrat selon la revendication 2, dans lequel X¹ et X² sont tous les deux -O-, et n et m sont chacun des nombres entiers de 2 à 8.

5. Procédé de synthèse d'un ensemble de polymères divers sur un support solide ayant des espaceurs optionnels, lesdits polymères pouvant être retirés de celui-ci après un traitement avec un réactif de clivage disulfure adapté, ledit procédé comprenant :
(a) la mise en contact d'un support solide avec un groupe de liaison disulfure non symétrique de formule :
P¹-X¹-(W¹)ₙ-S-S-(W²)ₘ-X²-P² (IIb)
dans laquelle,
P¹ et P² sont chacun des membres choisis indépendamment dans le groupe constitué par un atome d'hydrogène, un groupe activateur et un groupe protecteur ;
X¹ et X² sont choisis chacun indépendamment dans le groupe constitué par une liaison, -O-, -NH-, -NR- et -CO₂-, dans laquelle R est un groupe alkyle inférieur ayant de un à quatre atomes de carbone ;
W¹ et W² sont choisis chacun indépendamment dans le groupe constitué par le méthylène, l'oxyéthylène et l'oxypropylène ;
et
n et m sont chacun indépendamment des nombres entiers de 2 à 12 à la condition que n et m ne soient pas les mêmes quand W¹ et W² sont les mêmes ;
pour produire un support solide dérivé ayant des groupes de liaison disulfure non symétriques attachés protégés de manière adaptée avec des groupes protecteurs ;
(b) éventuellement retirer lesdits groupes protecteurs dudit support solide dérivé pour fournir un support solide dérivé ayant des groupes de liaison disulfure non symétriques avec des sites d'initiation de synthèse ; et
(c) le couplage desdits polymères auxdits sites d'initiation de synthèse sur ledit support solide dérivé pour produire un support solide ayant attaché un ensemble de polymères divers que l'on peut retirer de celui-ci après l'application dudit réactif de clivage disulfure.

6. Procédé de synthèse d'un ensemble de polymères divers sur un substrat, comprenant :
(a) la fourniture d'un substrat modifié pour une utilisation dans la synthèse chimique en phase solide, ledit substrat ayant la formule :
A¹-B¹-L¹ (II)
dans laquelle A est un support solide, B est une liaison ou un groupe espaceur, et L est un groupe de liaison ayant la formule :
P¹-X¹-(W¹)ₙ-S-S-(W²)ₘ-X²- (IIa)
dans laquelle :
P¹ est un groupe protecteur ;
X¹ et X² sont choisis chacun indépendamment dans le groupe constitué par une liaison, -O-, -NH-, -NR- et -CO₂-, dans laquelle R est un groupe alkyle inférieur ayant de un à quatre atomes de carbone ;
W¹ et W² sont choisis chacun indépendamment dans le groupe constitué par le méthylène, l'oxyéthylène et l'oxypropylène ;
et
n et m sont chacun indépendamment des nombres entiers de 2 à 12 à la condition que n et m ne soient pas les mêmes quand W¹ et W² sont les mêmes ; et
(b) la préparation d'un ensemble de polymères divers sur ledit substrat modifié.

7. Procédé selon la revendication 6, dans lequel P¹ est un groupe protecteur photolabile.

8. Procédé selon la revendication 6, dans lequel P¹ est un groupe protecteur photolabile, W¹ et W² sont tous les deux un méthylène, et X¹ et X² sont tous les deux -O-.

9. Procédé selon la revendication 6, dans lequel P¹ est un groupe protecteur photolabile, X¹ et X² sont tous les deux -O-, et n et m sont chacun des nombres entiers de 2 à 8.

10. Procédé selon la revendication 6, dans lequel P¹ est le DMT, X¹ et X² sont tous les deux -O-, W¹ et W² sont tous les deux un méthylène; et n et m sont chacun des nombres entiers de 2 à 8.

11. Procédés selon les revendications 9 ou 10, dans lesquels n vaut 2.

12. Procédé selon la revendication 6, dans lequel les polymères sont des peptides.

13. Procédé selon la revendication 6, dans lequel les polymères sont des polynucléotides.

14. Procédé selon la revendication 6, dans lequel la préparation d'un ensemble comprend : (i) le retrait éventuel desdits groupes protecteurs dudit substrat modifié pour fournir un support modifié avec des sites d'initiation de synthèse ; et
(ii) le couplage de monomères auxdits sites d'initiation de synthèse sur ledit substrat modifié pour produire un substrat modifié ayant un ensemble de polymères divers.

15. Procédé selon la revendication 14, dans lequel les régions sélectionnées du support modifié sont activées par de la lumière.

16. Procédé selon la revendication 6, comprenant en outre la libération des polymères du support modifié.
